# EUROPEAN PATENT SPECIFICATION

(11) **EP 3 006 013 B1**
(45) Date of publication and mention of the grant of the patent: **30.05.2018**
(21) Application number: 14808461.9
(22) Date of filing: 05.06.2014
(51) Int. Cl.: A61K 8/19, A61K 8/25, A61Q 11/00

(54) **ORAL COMPOSITION CONTAINING DIAMOND PARTICLES**
ORALE ZUSAMMENSETZUNG MIT DIAMANTPARTIKELN
COMPOSITION ORALE CONTENANT DES PARTICULES DE DIAMANT

(30) Priority: 07.06.2013 JP 2013120779
(43) Date of publication of application: 13.04.2016
(73) Proprietor: Sunstar Suisse SA, 1163 Etoy (CH)
(72) Inventor: FUJISAWA, Kouichi, 1163 Etoy (CH)
(74) Representative: TBK
(86) International application number: PCT/JP2014/064934
(87) International publication number: WO 2014/196592

(56) References cited:
- WO-A2-2010/060653
- JP-A- H1 017 443
- JP-A- 2004 238 321
- JP-A- 2007 091 636
- JP-A- 2012 509 920
- US-A1- 2009 130 031
- US-A1- 2010 254 915
- US-A1- 2010 254 915
- US-B1- 6 399 111
- 'SIDENT' vol. 9, March 2013, XP055299184 Retrieved from the Internet: <URL:http: //www.sipernat.com/product/sipernat/Documen ts/ SIDENT-9-EN.pdf> [retrieved on 2014-08-08]

## Description

### Technical Field

The present invention relates to an oral composition comprising diamond particles, and more specifically to a composition for the oral cavity, comprising specific diamond particles and specific abrasive silica particles. This application claims priority to Japanese Patent Application No. 2013-120779 filed on June 7, 2013.

### Background Art

Tooth discoloration results from deposition on the teeth of chromogenic substances called "stains." This causes a serious problem in terms of aesthetics. As a means for removing stains, tooth brushing is important. For example, an oral composition such as a dentifrice composition comprising a dental abrasive is held in the mouth with a tool such as a toothbrush, and the teeth are brushed in order to remove stains from the surfaces of the teeth. A dental abrasive with higher abrasive power is considered to have a higher stain removal effect and thus appears to be preferable to achieve this purpose. This is because a harder dental abrasive causes abrasion on the tooth surface with greater physical strength and is thus considered to provide a higher stain removal effect.

However, when an oral composition containing a dental abrasive that has high abrasive power is used, there is a risk that more abrasion than necessary may be caused on the teeth. For example, alumina, which is a typical example of a hard dental abrasive, has excellent stain removal power, but also damages the teeth by abrasion. Although various strategies have been developed to prevent damage to the teeth, the problem has not yet been solved. Today, oral compositions rarely contain alumina.

US2010254915 discloses a whitening dentifrice or tooth polishing composition comprising diamond particles 0.05 to 5.0 microns. The most preferred range of sizes is 0.1 to 0.5 microns. All the examples use diamond powder with a size of 0.5 microns. The examples also all comprise a silica abrasive.

WO2010060653 discloses an oral care product comprising diamond particles having sizes within a first size range of 0.3 to 2.5 microns. These block tubules and also remove stains and polish teeth.

RDA (relative dentin abrasion) and REA (relative enamel abrasion) are widely used as indices of the abrasive power of dental abrasives. As defined, RDA is an index of the degree of abrasion of tooth dentin, whereas REA is an index of the degree of abrasion of tooth enamel. The surface of teeth is enamel. However, when gums recede due to periodontitis or the like, dentin may be exposed. Therefore, RDA and REA should both be measured.

To remove stains or the like from the tooth surface, dental abrasives preferably have at least some level of RDA. However, dental abrasives with RDA that is too high may cause too much abrasion on the surface of teeth, and are thus not preferable. The 1995 ISO international standards on "physical properties and safety of dental abrasives" state that RDA should be not higher than 250. Dentin is usually not exposed on the surface. Even if dentin is exposed, damage to dentin should be minimized. Accordingly, REA, which is an index of the wear of enamel normally exposed on the tooth surface, preferably does not exceed a specific level (typically 40).

As described above, there is a need for an oral composition that has high stain removal power and appropriate abrasive power.

### Citation List

### Patent Literature

PTL 1: WO 10/060653 pamphlet

### Summary of Invention

### Technical Problem

An object of the present invention is to provide an oral composition that has great ability to remove stains from the tooth surface and appropriate abrasive power.

### Solution to Problem

The present inventors found that an oral composition comprising specific diamond particles and specific abrasive silica particles has remarkably high stain removal power as well as abrasiveness such that its abrasive power on dentin is not more than the optimal value (about 150) and its abrasive power on enamel is neither too small nor too great. The inventors completed the present invention with further improvements based on this finding.

Specifically, the present invention encompasses, for example, the subjects described in the following items.
Item 1. An oral composition comprising diamond particles and abrasive silica particles, the diamond particles having a median size (d50) of 2.0 to 3.5 µm, and the abrasive silica particles having an RDA of not less than 50 and not more than 200.
Item 2. The oral composition according to item 1, wherein the diamond particles are such that the difference between the 10th percentile value (d10) in cumulative particle size distribution and d50 is not more than 2 µm, and the difference between the 99th percentile value (d99) in the cumulative particle size distribution and d50 is not more than 3 µm.
Item 3. The oral composition according to Item 1 or 2, wherein the abrasive silica particles have a median size (d50) of 2.5 to 10 µm.
Item 4. The oral composition according to any one of Items 1 to 3, wherein the diamond particles are present in an amount of 0.1 to 0.3 mass%.
Item 5. The oral composition according to any one of Items 1 to 4, wherein the abrasive silica particles are present in an amount of 1 to 10 mass%.
Item 6. The oral composition according to any one of Items 1 to 5, further comprising a sulfosuccinate surfactant.

### Advantageous Effects of Invention

The oral composition of the present invention has excellent stain removal power and excellent abrasive power. (That is, the abrasive power on dentin is not higher than the optimal value (preferably not higher than about 150) and the abrasive power on enamel is neither too small nor too large). Therefore, the oral composition of the present invention can be preferably used for dental care.

### Description of Embodiments

The present invention is described below in more detail.

The oral composition of the present invention comprises specific diamond particles and specific abrasive silica particles.

The specific diamond particles have a median size (particle diameter corresponding to 50% in the cumulative particle size distribution (i.e., 50% cumulative diameter): d50) of 2 to 3.5 µm, preferably 2.5 to 3.5 µm, and more preferably 2.5 to 3 µm. The diamond particles preferably have a sharp particle size distribution. Specifically, the diamond particles are preferably such that the difference between the 10th percentile value (10% cumulative diameter: d10) in the cumulative particle size distribution and d50 is not more than 2 µm, and the difference between the 99th percentile value (99% cumulative diameter: d99) in the cumulative particle size distribution and d50 is not more than 3 µm. The difference between d10 and d50 is more preferably not more than 1.5 µm, and even more preferably not more than 1 µm. The difference between d99 and d50 is more preferably not more than 2.5 µm, and even more preferably not more than 2 µm.

The particle size (cumulative diameter) of the diamond particles referred to herein is a value determined based on the Coulter principle (the electrical sensing zone method) using water as a dispersant (i.e., a value obtained using a Coulter counter). This value can be measured, for example, by using a Multisizer 3 Coulter Counter (produced by Beckman Coulter, Inc.).

The particle size (cumulative diameter) of the silica particles referred to herein is a value obtained by using laser diffraction scattering. This value can be measured, for example, by using a Malvern Mastersizer based on the principle of Fraunhofer diffraction using a low-power He/Ne laser.

There is not much difference in effect regardless of whether the diamond is natural or synthetic. Synthetic diamonds, which are available at a relatively low price, are preferable. Such diamond particles can be obtained by purchasing commercially available products from Micro-diamond Co., Ltd., BM Group Co., Ltd., etc.

The present inventors found that an oral composition containing diamond particles has specific properties such that the composition has some level of cleaning power (PCR), although insufficient, and causes good abrasion on the hard part (enamel) of the tooth surface, while it does not cause much abrasion on the soft part (dentin) of the tooth surface. Accordingly, when diamond particles are incorporated in an oral composition, the REA of the resulting composition may be too high. To ensure sufficient cleaning properties, silica with an RDA value of not lower than a specific level (50 or higher) should also be used. However, if silica is incorporated in an amount sufficient to ensure cleaning properties in terms of stain removal, the RDA of the dentifrice becomes too high, and daily use of such a dentifrice may damage the teeth. The present inventors found that combined use of abrasive silica with diamond having a specific particle diameter and a specific particle size distribution can provide appropriate REA, RDA, and PCR.

The REA of the dentifrice (diamond particles and abrasive silica particles) referred to herein is a value determined in accordance with the method described in ISO 11609: 2010 Annex B. The REA can be obtained, for example, by requesting Therametric Technologies, Inc., Indiana 46060, USA, to perform the measurement.

In general, the dental abrasive preferably has an REA of 40 or less.

The specific abrasive silica particles have an RDA value of at least 50 and not more than 200, preferably at least 75 and not more than 170, and more preferably at least 100 and not more than 160, and most preferably at least 120 and not more than 150. The method for producing abrasive silica particles is not particularly limited. Such silica particles may be, for example, precipitated silica particles, fused silica particles, and the like.

The RDA of the particles as referred to herein is a value determined by the method described in ISO 11609: 2010 Annex B. The RDA can be obtained, for example, by requesting Therametric Technologies, Inc., Indiana 46060, USA, to perform the measurement.

The abrasive silica particles preferably have a median size (50% cumulative diameter; d50) of 2.5 to 10 µm, and more preferably 2.5 to 9 µm. A median size that is too large is not preferable because the PCR value does not increase and the RDA value increases.

As such abrasive silica particles, for example, commercial products available from PQ Silicas, Ltd., Huber Corporation, Evonik Degussa, and Rhodia, Inc., can be purchased and used.

The oral composition of the present invention preferably comprises the specific diamond particles in an amount of 0.1 to 0.3 mass%, and more preferably 0.15 to 0.25 mass%. Such levels of the diamond particle content can more preferably provide the effect of the present invention and also preferably inhibit the REA from becoming too high.

The oral composition of the present invention preferably comprises the specific abrasive silica particles in an amount of at least 1 mass% and not more than 10 mass%, and more preferably 1 to 8 mass%, and even more preferably 2 to 7 mass%. Such levels of the abrasive silica content can more preferably provide the effect of the present invention.

In particular, an oral composition having the above diamond particle content and the above abrasive silica particle content can particularly preferably provide the effect of the present invention.

Preferably, the oral composition of the present invention preferably further comprises a sulfosuccinate surfactant. The sulfosuccinate surfactant is a component usually incorporated as a foaming agent into compositions for the oral cavity. An oral composition of the present invention that contains a sulfosuccinate surfactant in addition to diamond particles and abrasive silica particles can synergistically produce the effect of the present invention more preferably.

Although the sulfosuccinate surfactant that can be used in the present invention is not particularly limited, alkyl sulfosuccinate surfactants are preferable. For example, sulfosuccinic acid monoesters can be preferably used. For example, the sulfosuccinic acid monoesters disclosed in JP2003-81796A can be used. Specifically, sulfosuccinic acid monoesters represented by formula (1) can be preferably used. 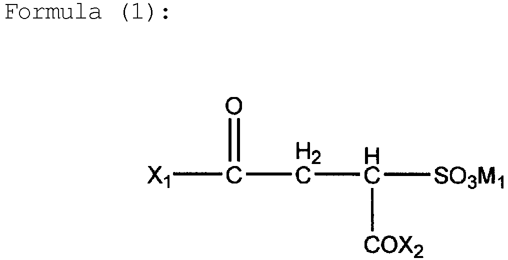 (wherein either X₁ or X₂ is R₁O-(AO)ₙ- or R₁CO-B-(AO)ₙ-, and the other is M₂O-; M₁ and M₂ are the same or different and each represents hydrogen, alkali metal, alkaline earth metal, ammonium, or alkanolamine; R₁ is an alkyl or alkenyl group having 8 to 22 carbon atoms; AO is an oxyalkylene group having 2 or 3 carbon atoms and the average addition mole number n of AO is 0 to 20; and B is -NH- or a monoalkanolamine residue having 2 or 3 carbon atoms.

In the sulfosuccic acid monoester represented by formula (1), R₁ is a naturally derived or synthetic linear or branched alkyl or alkenyl group having 8 to 22 carbon atoms, such as lauryl, cocoyl, myristyl, stearyl, synthetic C₁₂-C₁₄ alkyl, isononyl, isododecyl, octenyl, and dodecenyl. The longer the carbon chain of R₁, the more reduced is the bitterness and stimulus, whereas the shorter the carbon chain of R₁, the higher the effect of inhibiting stain formation. Thus, R₁ preferably has about 10 to 16 carbon atoms, and more preferably about 12 to 14 carbon atoms. In particular, synthetic C₁₂-C₁₄ synthetic alkyl or a combination of lauryl and myristyl is the most preferable.

M₁ and M₂ may be the same or different, and each represents hydrogen, alkali metal, alkaline earth metal, ammonium, or alkanolamine. Examples of alkali metals include sodium, potassium, and the like. Examples of alkali earth metals include magnesium and the like. Examples of alkanolamines include monoethanolamine, diethanolamine, triethanolamine, and the like. Among these, M₁ and M₂ are preferably sodium and magnesium, and are particularly preferably sodium.

The AO group is an oxyalkylene group having about 2 or 3 carbon atoms, and is preferably an oxyethylene group. The average addition mole number n of AO is preferably about 0 to 20. As the average addition mole number n decreases, the effect of inhibiting stain formation increases, and bitterness also decreases. Therefore, n is preferably about 0 to 7, and most preferably about 0 to 2. An average addition mole number of AO of 0 means that the sulfosuccinate monoester contains no oxyalkylene groups.

Examples of sulfosuccic acid monoesters represented by formula (1) are disodium polyoxyethylene (7 moles) lauryl sulfosuccinate, disodium polyoxyethylene (2 moles) lauryl sulfosuccinate, disodium polyoxyethylene (1 mole) lauryl sulfosuccinate, disodium lauryl sulfosuccinate, disodium polyoxyethylene (7 moles) myristyl sulfosuccinate, disodium polyoxyethylene (2 moles) alkyl (C₁₂₋₁₄) sulfosuccinate, disodium polyoxyethylene (1 mole) alkyl (C₁₂₋₁₄) sulfosuccinate, disodium alkyl (C₁₂₋₁₄) sulfosuccinate, magnesium polyoxyethylene (2 moles) lauryl sulfosuccinate, magnesium polyoxyethylene (2 moles) alkyl (C₁₂₋₁₄) sulfosuccinate, ditriethanolamine polyoxyethylene (7 moles) myristyl sulfosuccinate, and the like.

The most suitable sulfosuccinic acid monoester is a sodium salt of formula (1) in which R₁ is an alkyl group having about 12 to 14 carbon atoms, the AO group is oxyethylene, and the average addition mole number n of AO is about 0 to 2. Specific examples are disodium polyoxyethylene (2 moles) alkyl (C₁₂₋₁₄) sulfosuccinate, disodium polyoxyethylene (1 mole) alkyl (C₁₂₋₁₄) sulfosuccinate, disodium alkyl (C₁₂₋₁₄) sulfosuccinate, and the like.

Sulfosuccinate surfactants can be used singly or in combination of two or more.

The oral composition of the present invention preferably comprises a sulfosuccinate surfactant in an amount of, for example, about 0.1 to 10 mass%, more preferably about 0.2 to 5 mass%, and even more preferably about 0.5 to 2 mass%.

The oral composition of the present invention can be produced by using a known method. The oral composition of the present invention can be used for teeth and dentures, and can be formed into dentifrices in various forms by usual methods, such as paste dentifrices, powder dentifrices, cream dentifrices, gel dentifrices, liquid dentifrices, pastes, and the like. In particular, paste dentifrices, powder dentifrices, cream dentifrices, or gel dentifrices are preferable.

The oral composition of the present invention can be produced, for example, by mixing diamond particles and silica particles (and other components, if necessary) with a base material that is pharmaceutically acceptable or hygienically acceptable in the oral cavity. Examples of such base materials include water, glycerol, ethylene glycol, diethylene glycol, polyethylene glycol, propylene glycol, polypropylene glycol, sorbitol, xylitol, lactitol, mannitol, ethanol, and the like.

The oral composition of the present invention may contain other components (optional components) that are usually incorporated into oral compositions.

Examples of surfactants that can be incorporated include, in addition to alkyl sulfosuccinate surfactants, nonionic surfactants, anionic surfactants, and amphoteric surfactants. Specific examples of nonionic surfactants include fatty acid esters, fatty acid alkanolamides, sorbitan fatty acid esters, fatty acid monoglycerides, polyglycerol fatty acid esters, polyoxyethylene alkyl phenyl ethers, alkyl glycosides, diethyl sebacate, polyoxyethylene hydrogenated castor oils, polyoxyethylene sorbitan fatty acid esters, and the like. Examples of anionic surfactants include alkyl sulfates (such as sodium lauryl sulfate), polyoxyethylene alkyl ether sulfates, polyoxyethylene alkyl ether sulfosuccinates, N-acylamino acid salts, N-acyltaurine salts, alkyl ether carboxylates, alkyl phosphates, polyoxyethylene alkyl ether phosphates, fatty acid monoglyceride sulfates, alkyl sulfoacetates, and the like. Examples of amphoteric surfactants include alkyl dimethyl aminoacetate betaines, alkyl amidopropyldimethyl aminoacetate betaines, N-acyl-N-carboxymethyl-N-hydroxyethylethylenediamines, N-alkylaminoethylglycines, and the like. These surfactants can be used singly or in a combination of two or more. Such surfactants are typically incorporated in an amount of 0.1 to 10% by mass, based on the total mass of the composition.

Examples of thickeners that can be incorporated include cellulose derivatives such as carrageenan, carboxymethyl cellulose or salts thereof, hydroxypropyl cellulose, hydroxypropyl methyl cellulose, hydroxyethyl cellulose, and crystalline cellulose-carmellose sodium; gums such as xanthan gum, tragacanth gum, karaya gum, gum arabic, and gellan gum; synthetic binders such as polyvinyl alcohol, sodium polyacrylate, carboxy vinyl polymer, and polyvinylpyrrolidone; inorganic binders such as thickening silica (e.g., silica having an RDA of 10 or less), silylated silica, aluminum silica gel, and veegum; sodium alginate, pectin, soybean polysaccharides, sodium chondroitin sulfate, sodium hyaluronate, and the like. These thickeners can be used singly or in a combination of two or more. Such thickeners can be typically incorporated in an amount of 0.01 to 20% by mass.

Examples of flavoring agents that can be incorporated include menthol, carboxylic acid, anethole, eugenol, methyl salicylate, limonene, ocimene, n-decyl alcohol, citronellal, α-terpineol, methyl acetate, citronellyl acetate, methyleugenol, cineol, linalool, ethyl linalool, thymol, spearmint oil, peppermint oil, lemon oil, orange oil, sage oil, rosemary oil, cinnamon oil, beefsteak plant oil, wintergreen oil, clove oil, eucalyptus oil, pimento oil, d-camphor, d-borneol, fennel oil, cinnamon oil, cinnamaldehyde, mint oil, vanillin, and the like. These flavoring agents can be used singly or in a combination of two or more. Such flavoring agents can be typically incorporated in an amount of 0.01 to 1% by mass, based on the total mass of the composition.

Examples of sweetening agents that can be incorporated include saccharin sodium, acesulfame potassium, stevioside, stevia extract, sucralose, neohesperidyl dihydrochalcone, glycyrrhizin, perillartine, thaumatin, asparatyl phenylalanyl methyl ester, p-methoxycinnamic aldehyde, and the like. These sweetening agents can be used singly or in a combination of two or more. Such sweetening agents can be typically used in an amount of 0.01 to 1% by mass, based on the total mass of the composition.

Examples of wetting agents that can be incorporated include sorbitol, ethylene glycol, propylene glycol, glycerol, 1,3-butylene glycol, polypropylene glycol, xylitol, maltitol, lactitol, Palatinit, polyethylene glycol, and the like. Such wetting agents can be used singly or in a combination of two or more.

Examples of preservatives that can be incorporated include parabens such as methylparaben, ethylparaben, propylparaben, and butylparaben; sodium benzoate, phenoxyethanol, alkyldiaminoethylglycine hydrochloride, and the like. These preservatives can be added singly or in a combination of two or more.

Examples of colorants that can be incorporated include legal colors, such as Blue No. 1, Yellow No. 4, Red No. 202, and Green No. 3; mineral-based pigments, such as ultramarine blue, deep ultramarine blue, and Prussian blue; titanium oxide; and the like. These colorants can be used singly or in a combination of two or more.

Examples of pH adjusters that can be used include citric acid, phosphoric acid, malic acid, pyrophosphoric acid, lactic acid, tartaric acid, glycerophosphoric acid, acetic acid, nitric acid, chemically acceptable salts thereof, sodium hydroxide, and the like. These pH adjusters can be used singly or in a combination of two or more so that the composition has a pH of 4 to 8, and more preferably 5 to 7. Such pH adjusters can be used, for example, in an amount of 0.01 to 2 wt.%.

The oral composition of the present invention may contain, as a medicinal ingredient, cationic disinfectants such as cetylpyridinium chloride, benzalkonium chloride, benzethonium chloride, chlorhexidine hydrochloride, and chlorhexidine gluconate; vitamin E such as dl-α-tocopherol acetate, tocopherol succinate, and tocopherol nicotinate; amphoteric disinfectants such as dodecyldiaminoethylglycine; nonionic disinfectants such as triclosan and isopropylmethylphenol; enzymes such as dextranase, amylase, protease, mutanase, lysozyme, and lytic enzymes; fluorides such as sodium monofluorophosphate, sodium fluoride, and stannous fluoride; tranexamic acid, epsilon aminocaproic acid, aluminum chlorohydroxy allantoin, dihydrocholesterol, glycyrrhetinic acid, glycerophosphate, chlorophyll, sodium chloride, calpeptide, dipotassium glycyrrhizinate, allantoin, hinokitiol, potassium nitrate, and the like. These components can be added singly or in a combination of two or more.

There is no specific limitation on the container for holding the oral composition of the present invention. For example, a container made of glass, metal, plastic, a laminate material, or the like can be used. The shape of the container is also not particularly limited. For example, a container such as a bottle, a cup, a pouch, or a tube can be used.

### Examples

The present invention is described below in more detail with reference to Examples but is not limited to these.

### Preparation of the Oral Composition

Oral compositions (pastes) in Examples 1 to 9 and Comparative Examples 1 to 3 were prepared according to the formulations shown in Table 1 below. Specifically, after starting materials other than diamond particles, abrasive silica particles, and foaming agents (sodium sulfosuccinate and sodium lauryl sulfate) were mixed, the diamond particles and abrasive silica particles were added, and the resulting mixture was stirred and homogenized. Lastly, the foaming agents were added and the resulting mixture was stirred and homogenized. (This process also functioned as a defoaming treatment.) The diamond particles used were purchased from Microdiamant AG, CH-8574 Lingwil, Switzerland. All numerals for the components in Table 1 are by mass%.

Five types of commercially available abrasive precipitated silica particles (silica A to silica E), and abrasive fused silica (silica F) were used as abrasive silica particles. The median size (d50) and RDA of each of the abrasive silica particles were as follows.
(Silica particles A) d50: 2 µm, RDA: 120
(Silica particles B) d50: 3 µm, RDA: 160
(Silica particles C) d50: 4 µm, RDA: 200
(Silica particles D) d50: 8 µm, RDA: 150
(Silica particles E) d50: 14 µm, RDA: 100
(Silica particles F) d50: 14 µm, RDA: 130

Three types of diamond particles (diamond particles A to C) were used. Diamond particles A had a d50 of 2.8 µm, a d10 of 1.8 µm, and a d99 of 4.5 µm. Diamond particles B had a d50 of 2 µm, a d10 of 1 µm or more, and a d99 of 3.5 µm or less. Diamond particles C had a d50 of 3 µm, and a d10 of 1.8 µm or more, and a d99 of 4.5 µm or less.

Among the components used, thickening silica belongs to precipitated silica, but has an RDA of 10 or less, and does not have the abrasive power required of compositions for the oral cavity. Thickening silica is used as a thickener, not as abrasive silica. The thickening silica used has an average particle size of 13 µm.

The particle size of the abrasive silica particles mentioned above is determined by using a Malvern Mastersizer based on the principle of Fraunhofer diffraction using a low-power He/Ne laser. The particle size of the diamond particles above is determined by using Coulter Counter Multisizer 3 (produced by Beckman Coulter, Inc.). The RDA above is determined in accordance with the method described in ISO 11609: 2010 Annex B.

### Evaluation of Oral Composition

The stain removal power (PCR; pellicle cleaning ratio) of each of the obtained oral compositions was determined according to the method of Stooky (J Dent Research, 61: 1236-1239, 1982). RDA was evaluated according to the method disclosed in ISO 11609: 2010 Annex B. Table 1 shows the results.

Using diamond particles having a different median size (median size: 5 µm or 10 µm), oral compositions having the same formulation as in Comparative Example 1 were prepared and termed "Comparative Example 1a" (using diamond particles with a median size of 5 µm) and "Comparative Example 1b" (using diamond particles with a median size of 10 µm). The REA of the oral compositions in these Comparative Examples was measured in accordance with the method described in ISO 11609: 2010 Annex B, except that enamel was used in place of dentin, the number of reciprocal strokes was changed from 1,500 to 10,000, and the radiation dose was changed to 10. The results show that the oral composition obtained in Comparative Example 1a had an REA of about 45, and the oral composition obtained in Comparative Example 1b had an REA of about 100. The REA values of the compositions obtained in Example 5 and Comparative Examples 1 to 5 were also measured in the same manner. The oral composition of Example 5 had an RDA value of 22.5, and the oral compositions of Comparative Examples 1, 2, 3, 4, and 5 had REA values of 21.4, 16.2, 28.0, 5.4, and 3.7, respectively.

The results of Comparative Examples 1 to 3 in the present application show the following. Although the RDA is generally often positively correlated with PCR, comparison between the compositions of Comparative Examples 1 to 3, which have similar RDA values in the range of about 15 to 20 as well as similar d10 values and similar d99 values, shows that diamond particles having a d50 of 2.8 µm to 3 µm have a higher PCR value than diamond particles having a d50 of 2 µm. On the other hand, the REA value tends to increase as the d50 value increases. These results suggest that the diamond particles to be used preferably have a d50 value of about 2 to 3.5 µm, and more preferably 2.5 to 3 µm.

The above results confirm that the oral composition according to the present invention has remarkably high stain-removal power as well as abrasiveness such that its abrasion on dentin is not too great and its abrasive power on enamel is neither too small nor too large.

**Table 1**

| | Examples | | | | | | | | | Comparative Examples | | | | |
|---|---|---|---|---|---|---|---|---|---|---|---|---|---|---|
| Formulation No. | 1 | 2 | 3 | 4 | 5 | 6 | 7 | 8 | 9 | 1 | 2 | 3 | 4 | 5 |
| Sorbitol solution (70%) | 50 | 50 | 50 | 50 | 50 | 50 | 50 | 50 | 50 | 50 | 50 | 50 | 50 | 50 |
| Saccharin sodium | 0.1 | 0.1 | 0.1 | 0.1 | 0.1 | 0.1 | 0.1 | 0.1 | 0.1 | 0.1 | 0.1 | 0.1 | 0.1 | 0.1 |
| Glycerol | 17 | 17 | 17 | 17 | 17 | 17 | 17 | 17 | 17 | 17 | 17 | 17 | 17 | 17 |
| Xanthan gum | 0.6 | 0.6 | 0.6 | 0.6 | 0.6 | 0.6 | 0.6 | 0.6 | 0.6 | 0.6 | 0.6 | 0.6 | 0.6 | 0.6 |
| Sodium carboxymethyl cellulose | 0.5 | 0.5 | 0.6 | 0.5 | 0.6 | 0.6 | 0.6 | 0.5 | 0.5 | 0.5 | 0.5 | 0.5 | 0.5 | 0.5 |
| Thickening silica | 12 | 12 | 12 | 12 | 12 | 12 | 12 | 12 | 12 | 12 | 12 | 12 | 12 | 12 |
| Titanium oxide | 0.6 | 0.6 | 0.6 | 0.6 | 0.6 | 0.6 | 0.6 | 0.6 | 0.6 | 0.6 | 0.6 | 0.6 | 0.6 | 0.6 |
| Methyl paraben | 0.1 | 0.1 | 0.1 | 0.1 | 0.1 | 0.1 | 0.1 | 0.1 | 0.1 | 0.1 | 0.1 | 0.1 | 0.1 | 0.1 |
| Propylene glycol | 3 | 3 | 3 | 3 | 3 | 3 | 3 | 3 | 3 | 3 | 3 | 3 | 3 | 3 |
| Sodium lauryl sulfate | 1.2 | 1.2 | 1.2 | 1.2 | 1.2 | 1.2 | 1.2 | 1.2 | 1.2 | 1.2 | 1.2 | 1.2 | 1.2 | 1.2 |
| POE disodium alkyl sulfosuccinate | 1 | 1 | 1 | 1 | 1 | 1 | 1 | 1 | 1 | 1 | 1 | 1 | 1 | |
| Diamond particle A | 0.2 | 0.2 | 0.2 | 0.2 | 0.2 | 0.2 | 0.2 | 0.2 | 0.2 | 0.2 | | | | |
| Diamond particle B | | | | | | | | | | | 0.2 | | | |
| Diamond particle C | | | | | | | | | | | | 0.2 | | |
| Silica particles A | 5 | 6 | | | | | | | | | | | | |
| Silica particles B | | | 4 | 5 | | | | | | | | | | |
| Silica particles C | | | | | 3 | 4 | 5 | | | | | | | |
| Silica particles D | | | | | | | | 4 | 5 | | | | | |
| Silica particles E | | | | | | | | | | | | | | 7.2 |
| Silica particles F | | | | | | | | | | | | | 2 | |
| Ion-exchanged water | Balance | Balance | Balance | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 | 100 | 100 | 100 |
| RDA | 112 | 119 | 123 | 145 | 127 | 145 | 176 | 104 | 116 | 15 | 13 | 17 | 55 | 46 |
| PCR | 111 | 118 | 118 | 120 | 111 | 116 | 118 | 118 | 113 | 61 | 55 | 72 | 80 | 68 |

## Claims

1. An oral composition comprising diamond particles and abrasive silica particles, the diamond particles having a median size (d50) of 2.0 to 3.5 µm, and the abrasive silica particles having an RDA of at least 50 and not more than 200.

2. The oral composition according to claim 1, wherein the diamond particles are such that the difference between the 10th percentile value (d10) in cumulative particle size distribution and d50 is not more than 2 µm, and the difference between the 99th percentile value (d99) in the cumulative particle size distribution and d50 is not more than 3 µm.

3. The oral composition according to claim 1 or 2, wherein the abrasive silica particles have a median size (d50) of 2.5 to 10 µm.

4. The oral composition according to any one of claims 1 to 3, wherein the diamond particles are present in an amount of 0.1 to 0.3 mass%.

5. The oral composition according to any one of claims 1 to 4, wherein the abrasive silica particles are present in an amount of 1 to 10 mass%.

6. The oral composition according to any one of claims 1 to 5, further comprising a sulfosuccinate surfactant.

## Patentansprüche

1. Orale Zusammensetzung, die Diamantteilchen und abrasive Siliciumdioxidteilchen umfasst, wobei die Diamantteilchen eine mediane Größe (d50) von 2,0 bis 3,5 µm aufweisen, und die abrasiven Siliciumdioxidteilchen eine RDA von wenigstens 50 und nicht mehr als 200 aufweisen.

2. Orale Zusammensetzung nach Anspruch 1, wobei die Diamantteilchen derartig sind, dass der Unterschied zwischen dem Wert der 10. Perzentile (d10) in der kumulativen Teilchengrößenverteilung und d50 nicht mehr als 2 µm ist, und der Unterschied zwischen dem Wert der 99. Perzentile (d99) in der kumulativen Teilchengrößenverteilung und d50 nicht mehr als 3 µm ist.

3. Orale Zusammensetzung nach Anspruch 1 oder 2, wobei die abrasiven Siliciumdioxidteilchen eine mediane Größe (d50) von 2,5 bis 10 µm aufweisen.

4. Orale Zusammensetzung nach einem der Ansprüche 1 bis 3, wobei die Diamantteilchen in einer Menge von 0,1 bis 0,3 Massen-% vorhanden sind.

5. Orale Zusammensetzung nach einem der Ansprüche 1 bis 4, wobei die abrasiven Siliciumdioxidteilchen in einer Menge von 1 bis 10 Massen-% vorhanden sind.

6. Orale Zusammensetzung nach einem der Ansprüche 1 bis 5, die außerdem ein Sulfosuccinat-Tensid umfassen.

## Revendications

1. Composition orale comprenant des particules de diamant et des particules de silice abrasive, les particules de diamant ayant une taille médiane (d50) de 2,0 à 3,5 µm, et les particules de silice abrasive ayant une RDA d'au moins 50 et de pas plus de 200.

2. Composition orale selon la revendication 1, dans laquelle les particules de diamant sont telles que la différence entre la valeur du 10ème percentile (d10) dans la distribution cumulative des tailles des particules et d50 n'est pas de plus de 2 µm, et la différence entre la valeur du 99ème percentile (d99) dans la distribution cumulative des tailles des particules et d50 n'est pas de plus de 3 m.

3. Composition orale selon la revendication 1 ou 2, dans laquelle les particules de silice abrasive ont une taille médiane (d50) de 2,5 à 10 µm.

4. Composition orale selon l'une quelconque des revendications 1 à 3, dans laquelle les particules de diamant sont présentes dans une quantité de 0,1 à 0,3 % en masse.

5. Composition orale selon l'une quelconque des revendications 1 à 4, dans laquelle les particules de silice abrasive sont présentes dans une quantité de 1 à 10 % en masse.

6. Composition orale selon l'une quelconque des revendications 1 à 5, comprenant en outre un tensioactif de sulfosuccinate.
